Europäisches Patentamt

European Patent Office

Office européen des brevets

(11) Veröffentlichungsnummer: **0 080 008**
A1

# (12) EUROPÄISCHE PATENTANMELDUNG

(21) Anmeldenummer: 82102877.6

(22) Anmeldetag: 03.04.82

(51) Int. Cl.³: **A 61 F 13/02,** A 61 L 15/06, C 09 J 7/04

(30) Priorität: 19.11.81 DE 3145796

(43) Veröffentlichungstag der Anmeldung: 01.06.83
Patentblatt 83/22

(84) Benannte Vertragsstaaten: CH DE FR GB IT LI SE

(71) Anmelder: **Firma Carl Freudenberg, Höhnerweg 2,
D-6940 Weinheim/Bergstrasse (DE)**

(72) Erfinder: **Krull, Manfred, Hüttengasse 49,
D-6901 Heiligkreuzsteinach (DE)**

(74) Vertreter: **Weissenfeld-Richters, Helga, Dr.,
Höhnerweg 2, D-6940 Weinheim/Bergstrasse (DE)**

(54) **Leicht ablösbares Heftpflaster.**

(57) Leicht ablösbares Heftpflaster, bestehend aus einem luftdurchlässigen Flächengebilde und einer darauf aufgebrachten Beschichtung aus einer daueraktiven Haftmaße, wobei die Beschichtung Durchbrechungen in dicht benachbarten Bereichen aufweist.

EP 0 080 008 A1

Anmelderin: Firma Carl Freudenberg, Weinheim

## Leicht ablösbares Heftpflaster

Die Erfindung betrifft ein leicht ablösbares Heftpflaster, bestehend aus einem luftdurchlässigen Flächengebilde und einer
darauf aufgebrachten Beschichtung aus einer daueraktiven Haftmasse.

0080008

Heftpflaster dienen bevorzugt zur Befestigung von Wundverbänden. Sie bestehen im allgemeinen aus einem luftdurchlässigen Flächengebilde aus textilen Fasern, beispielsweise aus einem Gewebe oder einem Vliesstoff, und auf ihrer Unterseite ist eine filmartige Schicht aus einem daueraktiven Klebstoff aufgetragen. Wechselwirkungen zwischen der Luft und der überklebten Hautzone werden hierdurch unterbunden.

Um trotz der ständig von der Haut abgesonderten Feuchtigkeits- und Fettmengen eine gute Haftung zu erzielen, ist es erforderlich, der Schicht eine gewisse Mindestdicke zu verleihen. Solcher Art ausgerüstete Heftplaster sind aus diesem Grunde häufig nur schwierig bzw. unter Schmerzen von der Haut abzulösen.

Der Erfindung liegt die Aufgabe zugrunde, ein leicht ablösendes Heftplaster zu entwickeln, das sich durch eine gute Haftfestigkeit auszeichnet.

Diese Aufgabe wird erfindungsgemäß bei einem Heftplaster der eingangs genannten Art dadurch gelöst, daß die Beschichtung Durchbrechungen in dicht benachbarten Bereichen aufweist. Durch die Durchbrechungen kann die überklebte Haut in eine Wechselwirkung zu der umgebenden Luft treten und abgesonderte Feuchtigkeit vermag infolgedessen ohne weiteres zu verdunsten. Diese Feuchtigkeit vermag infolgedessen nicht die Haftfestigkeit der Verbindung zu beeinträchtigen. Der absolute Bedarf an Haftmasse je Flächeneinheit läßt sich dadurch ohne eine Beeinträchtigung der Festigkeit gegenüber üblichen Ausführungen vermindern.

Beim Ablösen des Heftpflasters, das gewöhnlich durch ein einfaches Abziehen vorgenommen wird, ergibt sich durch die Durchbrechungen ein ständig verlagerter Kraftangriff an der Oberfläche der Haut. Die Hautreizung als solche ist dadurch vermindert und das Ablösen gestaltet sich weniger schmerzhaft als bei den bekannten Ausführungen.

Für ein weiter erleichtertes Ablösen kann das Heftpflaster von der Rückseite mit einer Flüssigkeit durchnäßt werden, beispielsweise mit einer Seifenlösung oder Alkohol. Diese Flüssigkeiten vermögen durch die Durchbrechungen hindurch bis in den Bereich der eigentlichen Klebezone vorzudringen und dort infolge der erhöhten Angriffsfläche eine durchgehende Ablösung zu bewirken. Das Heftpflaster läßt sich bei einer entsprechenden Vorgehensweise auch von Hautzonen mit einer stärkeren Behaarung schmerzfrei ablösen.

Nach einer vorteilhaften Ausgestaltung ist es vorgesehen, daß die Durchbrechungen ineinander übergehen, so daß die Haftmassenflächen voneinander isolierte Zonen bilden. Die Ablösbarkeit wird hierdurch wesentlich erleichtert.

Die Mindestbreite der Teilschichten der Beschichtung zwischen den Durchbrechungen soll etwa deren maximaler Breite entsprechen. Als vorteilhaft hat es sich bewährt, wenn die Mindestbreite der Durchbrechungen 0,5 bis 3 mm beträgt, bei einer größten Breite der Teilschichten von 0,5 bis 3 mm.

Die angewendete Beschichtung kann aus den einschlägig verwendeten Lösungsmittel- oder Dispersionsklebern bestehen, beispielsweise auf Acrylat- oder Kautschukbasis. Das Flächengewicht der Beschichtung soll etwa 30 bis 40 $g/m^2$ betragen.

Das Flächengebilde kann aus den einschlägig verwendeten textilen Werkstoffen bestehen, beispielsweise aus einem Vliesstoff, einem Gewebe oder einem Gewirke. Das Flächengewicht kann je nach Anwendungsfall in einem Bereich zwischen 30 und 300 $g/m^3$ variieren, wobei der Bereich von 40 bis 60 $g/m^2$ bevorzugt wird. Neben Stapel- oder Endlosfasern aus Zellwolle, Baumwolle, Polyamid oder Polyester können auch Polyurethanfasern zur Anwendung kommen, die sich durch eine besonders hohe Elastizität auszeichnen.

In der in der Anlage beigefügten Zeichnung ist eine beispielhafte Ausführung des vorgeschlagenen Heftpflasters in der Aufsicht von unten wiedergegeben. Das luftdurchlässige, textile Flächengebilde ist mit 1 bezeichnet. Die mit Haftmasse beschichteten Teilflächen mit 2.

0080008

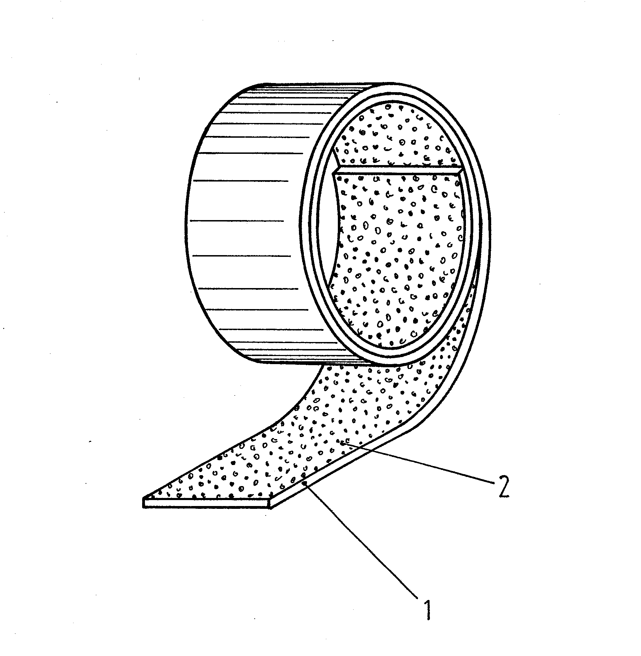

Patentansprüche

1. Leicht ablösbares Heftpflaster, bestehend aus einem luftdurchlässigen Flächengebilde und einer darauf aufgebrachten Beschichtung
aus einer daueraktiven Haftmasse, dadurch gekennzeichnet, daß die
Beschichtung Durchbrechungen in dicht benachbarten Bereichen
aufweist.

2. Heftpflaster nach Anspruch 1, dadurch gekennzeichnet, daß die
Durchbrechungen ineinander übergehen.

3. Heftpflaster nach Anspruch 2, dadurch gekennzeichnet, daß die
Mindestbreite der Teilschichten der Beschichtung zwischen den
Durchbrechungen etwa der maximalen Breite der Durchbrechungen
entspricht.

4. Heftpflaster nach Anspruch 2 bis 3, dadurch gekennzeichnet, daß
die Durchbrechungen eine Mindestbreite von 0,5 bis 3 mm haben.

5. Heftpflaster nach Anspruch 2 bis 4, dadurch gekennzeichnet, daß
die Teilschichten eine größte Breite von 0,5 bis 3 mm haben.

6. Heftpflaster nach Anspruch 1 bis 5, dadurch gekennzeichnet, daß
die Beschichtung aus einem Lösungsmittel- oder Dispersionskleber
besteht.

1/1

Europäisches
Patentamt

**EUROPÄISCHER RECHERCHENBERICHT**

0080008

Nummer der Anmeldung

EP 82 10 2877

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | DE-C- 974 178 (P. BEIERSDORF)<br>* Seite 2, Anspruch 1; Seite 2, Zeilen 46-49 * | 1 | A 61 F 13/02<br>A 61 L 15/06<br>C 09 J 7/04 |
| | --- | | |
| X | GB-A- 987 275 (JOHNSON & JOHNSON)<br>* Seite 5, Anspruch 1; Seite 1, Zeilen 11-14; Seite 3, Zeilen 10-23; Figuren 1-3 * | 1 | |
| | --- | | |
| A | FR-A-2 012 584 (SMITH & NEPHEW)<br>* Seite 31, Ansprüche 1,4,5 * | 1,6 | |
| | ----- | | |

|  | RECHERCHIERTE SACHGEBIETE (Int. Cl. ³) |
|---|---|
| | A 61 F<br>A 61 L<br>C 09 J |

Der vorliegende Recherchenbericht wurde für alle Patentansprüche erstellt.

| Recherchenort<br>DEN HAAG | Abschlußdatum der Recherche<br>03-03-1983 | Prüfer<br>GIRARD Y.A. |
|---|---|---|